# FASCICULE DE BREVET EUROPEEN

(11) **EP 2 207 608 B1**
(45) Date de publication et mention de la délivrance du brevet: **23.01.2019**
(21) Numéro de dépôt: 08858736.5
(22) Date de dépôt: 18.09.2008
(51) Int. Cl.: B01D 21/24, B01J 8/00

(54) **DISPOSITIF DE SEPARATION D'UN SOLIDE FINEMENT DIVISE EN SUSPENSION DANS UN LIQUIDE VISQUEUX**
VORRICHTUNG ZUM ABTRENNEN VON FEIN DISPERSEN FESTSTOFFEN AUS EINER VISKOSEN FLÜSSIGKEIT
DEVICE FOR SEPARATING A FINELY DIVIDED SOLID IN SUSPENSION IN A VISCOUS LIQUID

(30) Priorité: 18.10.2007 FR 0707291
(43) Date de publication de la demande: 21.07.2010
(73) Titulaire: IFP Energies nouvelles, 92500 Rueil-Malmaison (FR); ENI S.p.A., 00144 Rome (IT)
(72) Inventeur: VIGUIE Jean-Christophe, F-69006 Lyon (FR); NASTOL, Willi, F-38370 Les Roches de Condrie (FR); HUGUES, François, F-69390 Vernaison (FR); MIGNONE, Eisa, IT-20052 Monza (IT); BELMONTE, Giuseppe, IT-Torino (IT)
(74) Mandataire: IFP Energies nouvelles
(86) Numéro de dépôt international: PCT/FR2008/001305
(87) Numéro de publication internationale: WO 2009/074731

(56) Documents cités:
- WO-A-01/34266
- DE-A1- 2 209 349
- DE-A1- 4 432 280
- FR-A- 2 802 828
- GB-A- 170 225
- US-A- 2 593 036

## Description

### DOMAINE DE L'INVENTION

La présente invention se situe dans le domaine des dispositifs permettant d'effectuer la séparation par gravité de fines particules solides contenues dans un liquide plus ou moins visqueux. Ces dispositifs sont généralement connus sous le terme de décanteur. Ils permettent de récupérer en tête un liquide sensiblement débarrassé de particules, et en fond un liquide concentré en lesdites particules. Dans la suite du texte, nous parlons de liquide clair pour désigner le liquide extrait en tête du dispositif, et de liquide dense pour désigner le liquide concentré en particules solides soutiré en fond dudit dispositif.

On rencontre les décanteurs principalement dans l'industrie du traitement des boues et en raffinage pour le traitement des eaux chargées en huile.

Le dispositif selon la présente invention est plus particulièrement adapté à la décantation de particules solides ayant une taille comprise entre 10 et 100 microns (1 micron = 1 µm = 10⁻⁶ mètre), et de masse volumique (de la particule) comprise entre 500 kg/m3 et 2500 kg/m3.

Ces particules peuvent être de fines particules de catalyseur, telles par exemple que celles utilisées dans un réacteur de synthèse Fischer Tropsch opérant en phase liquide avec un catalyseur en suspension (appelé "slurry reactor" ou "slurry" selon la terminologie anglo-saxonne). Dans ce cas, les particules de catalyseur ont un diamètre compris entre 20 et 80 microns, une masse volumique comprise entre 1000 et 2500 kg/m3, et le milieu liquide dans lequel elles se trouvent dispersées a une viscosité comprise entre 0,2 et 2 mPa.s, de préférence entre 0,5 et 1 mPa.s, et une masse volumique comprise entre 500 et 1000 kg/m3.

L'unité de viscosité utilisé dans tout le texte est le milliPascal.seconde , soit 10⁻³ Pascal.s, qui est égal à 1 centipoise.

Dans la suite du texte, nous conserverons le terme de "slurry" pour désigner une suspension de fines particules dispersées dans un milieu liquide, le milieu liquide étant lui même traversé par des bulles de gaz.

### EXAMEN DE L'ART ANTERIEUR

Le dimensionnement des décanteurs repose essentiellement sur la notion de vitesse de sédimentation définie comme la vitesse de chute des particules dans le liquide considéré sous le seul effet de la gravité. Comme cette vitesse est d'autant plus faible que les particules sont de petite taille et de faible masse volumique, les décanteurs ont généralement des hauteurs importantes pour permettre aux fines particules de migrer vers le bas du dispositif où elles sont en généralement récupérées. Les décanteurs ont également des diamètres importants afin d'obtenir une vitesse linéaire d'écoulement faible entre l'entrée et la sortie du dispositif, de manière à perturber le moins possible la chute des particules dans le milieu considéré.

Nous examinons dans la suite l'art antérieur dans le domaine du procédé Fischer Tropsch, puisque celui ci constitue le cas d'application le plus fréquent du présent dispositif.

Le procédé Fischer Tropsch est un procédé de synthèse de molécules hydrocarbonées s'étendant approximativement de C1 à C80, à partir d'un gaz réactionnel appelé gaz de synthèse, essentiellement constitué d'hydrogène (H2) et de monoxyde de carbone (CO), et également de dioxyde de carbone (CO2).

Une des mises en oeuvre de ce procédé consiste à utiliser de fines particules de catalyseur à base de fer ou de cobalt, supporté ou non, et à les mettre en contact avec le milieu réactionnel constitué d'un mélange gaz-liquide, quelquefois appelé colonne à bulles.

Les particules ayant des diamètres compris entre 10 et 100 microns, et le plus souvent compris entre 20 et 80 microns, se trouvent dispersées au sein de la phase liquide elle même traversée par des bulles de gaz, l'ensemble du milieu ainsi constitué étant généralement appelé "slurry".

Un procédé Fischer Tropsch en "slurry" comporte généralement un premier dispositif permettant de séparer la majeure partie des gaz du liquide dans lequel se trouvent les particules de catalyseur. Le gaz ainsi libéré est essentiellement constitué d'hydrocarbures légers en C1 à C5, de la vapeur d'eau formée au cours de la réaction, ainsi que d'une fraction plus ou moins importante de gaz de synthèse n'ayant pas réagi.

Un second moyen de séparation situé en aval du premier moyen permet de séparer les particules de catalyseur de la phase liquide, cette dernière étant dirigée vers une zone de traitement en aval pour aboutir à la production de carburants, essentiellement essence, jet-fuel et gazole.

La phase contenant la majeure partie des particules solides (appelé liquide concentré) est généralement recyclée dans le réacteur de synthèse.

De manière générale, le milieu "slurry" dans un procédé Fischer Tropsch peut être chargé en particules de catalyseur jusqu'à des concentrations comprises entre 10% et 60% en poids, de préférence entre 20% et 45% en poids.

Les dispositifs de séparation des particules solides permettent de recycler au réacteur une suspension dont la concentration en particules solides est augmentée d'environ 10% en valeur relative, par rapport à la concentration dans la suspension à traiter.

Beaucoup des dispositifs utilisés dans l'art antérieur sont situés à l'intérieur même du réacteur de synthèse Fischer Tropsch.

Parmi les documents décrivant des dispositifs situés à l'extérieur du réacteur de synthèse, nous citerons:
- Le document US 5,770,629 qui décrit une zone de filtration externe, mais qui ne repose pas sur le principe de la décantation. Le dispositif selon la présente invention ne possède aucun élément de filtration.
- Le document WO 98/27181 qui décrit un décanteur muni d'un interne tubulaire permettant de définir une zone de recirculation annulaire utilisée pour la récupération du liquide clair. Le dispositif selon la présente invention ne possède aucune zone de recirculation.

Les documents DE 22 09 349 et GB 170 225 décrivent des décanteurs munis d'un interne tubulaire.

Le document FR 2 802 828 décrit un procédé Fischer Tropsch avec un dispositif de séparation de particules de catalyseur.

### DESCRIPTION SOMMAIRE DE LA FIGURE

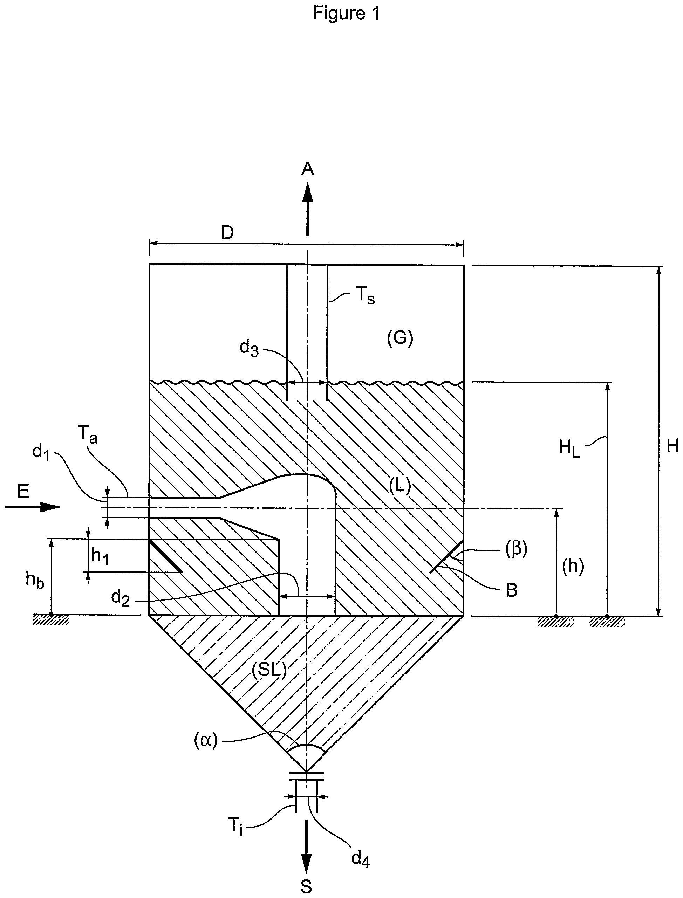

La figure 1 est une vue schématique du dispositif selon la présente invention dans lequel se trouvent rassemblés les principales caractéristiques de celui ci.

En allant de bas en haut, le premier volume (SL) correspond à la suspension concentrée en particules solides. Le second volume (L) correspond à la suspension comprenant liquide et solide, moins chargée en particules solides, et le troisième volume (G) situé au dessus du second volume (L) correspond à une zone essentiellement composée de gaz.

### DESCRIPTION SOMMAIRE DE L'INVENTION

La présente invention consiste en un dispositif permettant la séparation de fines particules de solide de diamètre généralement compris entre 10 et 100 microns, dispersées sous forme d'une suspension dans un liquide, ledit dispositif comportant une partie supérieure de forme cylindrique de rapport H/D compris entre 2 et 6, et une partie inférieure conique de même diamètre maximal que la partie cylindrique, l'angle du cône étant compris entre 40° et 60°, ledit dispositif comprenant en outre:
- une tubulure d'admission (Ta) de la suspension en forme de coude, la portion horizontale dudit coude de diamètre (d1) pénétrant à l'intérieur de la partie supérieure du dispositif, et la portion verticale dudit coude de diamètre (d2) débouchant sensiblement au niveau du plan séparant la partie supérieure et la partie inférieure du dispositif, le rapport des diamètres d2/d1 étant compris entre 2 et 4, et la portion verticale de la tubulure d'admission (Ta) ayant une longueur (hb) égale à au moins 3 fois le diamètre (d2) de l'extrémité de sortie de ladite portion verticale.
- une tubulure de soutirage supérieure (Ts) du liquide clair située au sommet de la partie cylindrique supérieure,
- une tubulure de soutirage inférieure (Ti) des particules solides située en bas de la partie conique inférieure.

La présente invention consiste également en un procédé de séparation de particules solide de diamètre compris entre 10 et 100 microns dispersées dans un liquide de viscosité supérieure à 0,2 mPa.s mettant en oeuvre le dispositif précédemment décrit.

L'invention s'applique de manière préférée à un procédé de synthèse Fischer Tropsch mettant en oeuvre un réacteur de synthèse Fischer-Tropsch en mode "slurry" avec un catalyseur constitué de fines particules de diamètre compris entre 10 et 100 microns en suspension dans un liquide de viscosité supérieure à 0,2 mPa.s, dans lequel ledit procédé utilise un dispositif selon l'invention situé à l'extérieur du réacteur de synthèse, et les particules extraites dudit dispositif par la tubulure inférieure (Ti) étant recyclées dans le réacteur au moyen d'une pompe.

### DESCRIPTION DETAILLEE DE L'INVENTION

Le dispositif selon la présente invention permet la séparation de particules de solide de diamètre compris entre 10 et 100 microns dispersées sous forme d'une suspension dans un liquide de viscosité supérieure à 0,2 mPa.s.

Ledit dispositif consiste en une partie supérieure de forme cylindrique de rapport H/D compris entre 2 et 6, et préférentiellement compris entre 2,5 et 5, et une partie inférieure conique de même diamètre maximal que la partie cylindrique, l'angle du cône (a) étant compris entre 40° et 60°, de préférence entre 45° et 55° .

Généralement le diamètre du décanteur selon l'invention est compris entre 1 et 10 mètres, préférentiellement compris entre 2 et 8 mètres, et de manière encore préférée compris entre 3 et 7 mètres.

Dans la suite du texte, on parlera indifféremment de partie supérieure ou cylindrique, et de partie inférieure ou conique.

Ledit dispositif comprend en outre:
- une tubulure d'admission (Ta) de la suspension en forme de coude, la portion horizontale dudit coude, de diamètre (d1), pénétrant à l'intérieur de la partie supérieure du dispositif, et la portion verticale dudit coude, de diamètre (d2), débouchant sensiblement au niveau du plan séparant la partie supérieure et la partie inférieure du dispositif, le rapport des diamètres d2/d1 étant compris entre 2 et 4,
- une tubulure de soutirage supérieure (Ts) du liquide clair située au sommet de la partie supérieure,
- une tubulure de soutirage inférieure (Ti) des particules solides située en bas de la partie inférieure.

On entend par sensiblement le fait que le débouché de la portion verticale de la tubulure d'admission (Ta) se fait au niveau du plan séparant la partie supérieure et la partie inférieure du dispositif à plus ou moins 0,5 mètre.

La tubulure d'admission (Ta) de la suspension a sa portion verticale (hb) dont la longueur est égale à au moins 3 fois le diamètre (d2) de l'extrémité de sortie de ladite portion verticale.

Selon une caractéristique préférée du dispositif selon l'invention, la tubulure de soutirage (Ts) du liquide clair possède une portion verticale sensiblement centrée sur l'axe de symétrie du décanteur et plongeant à une profondeur comprise entre 0,2 et 1 mètre au dessous du niveau dudit liquide clair (H_{L}), et préférentiellement à une profondeur comprise entre 0,3 et 0,7 mètre au dessous dudit niveau (H_{L}), pour des valeurs de H préférentiellement comprises entre 4 et 40 mètres, et de manière encore préférée comprises entre 6 et 30 mètres.

Selon une caractéristique préférée du dispositif, celui ci est muni d'un baffle intérieure (B) fixé à la paroi interne de la partie cylindrique dudit dispositif, et faisant un angle (β) par rapport à la verticale sensiblement identique au demi angle du cône (α) de la partie inférieure du dispositif. On entend par sensiblement un écart entre l'angle (β) et l'angle (α) inférieur à 10°.

Selon une autre caractéristique préférée, le baffle (B) est fixé à un niveau sensiblement identique au niveau où commence (dans le sens de haut en bas) la portion verticale de la tubulure d'admission (Ta).

On entend par "sensiblement" un niveau correspondant à celui du début de la portion verticale de la tubulure d'admission (Ta), ce niveau pouvant être compris dans un intervalle de + 0,3 m ou - 0,3 m par rapport audit niveau (hb) de la portion verticale.

La hauteur h1 du baffle (B) est comprise entre 0,4 et 0,6 fois la hauteur de la portion verticale de la tubulure d'admission (Ta) . La dite portion verticale est notée (hb) sur la figure 1.

La tubulure d'admission (Ta) pénètre à l'intérieur de la partie cylindrique du dispositif de manière sensiblement perpendiculaire à ladite paroi cylindrique. Le niveau de la portion horizontale de la tubulure d'admission (Ta) est précisément repéré comme le niveau de l'axe horizontal de ladite tubulure noté (h) sur la figure 1.

Le niveau de liquide (H_{L}) dans le dispositif peut être régulé par tout moyen connu de l'homme du métier tel qu'une mesure agissant sur le débit de liquide clair sortant (A), ou sur le débit de particules solides sortant (S), ou même sur le débit de la suspension à traiter (E).

De manière préférée, ce niveau liquide est choisi de manière que la tubulure de soutirage du liquide (Ts) clair soit enfoncée d'une profondeur comprise entre 0,3 et 0,7 m au dessous dudit niveau liquide (H_{L}).

Le dispositif selon la présente invention peut être utilisé dans un procédé dans lequel la vitesse d'écoulement du liquide dans la tubulure de soutirage (Ts) du liquide clair est généralement comprise entre 0,1 m/s et 0,3 m/s.

Le dispositif selon la présente invention peut également être utilisé dans un procédé dans lequel la vitesse de la suspension dans la tubulure de soutirage inférieure (Ti) des particules solide est généralement comprise entre 0,8 m/s et 3 m/s.

Le dispositif selon la présente invention peut être utilisé dans un procédé de séparation de fines particules de diamètre compris entre 10 et 100 microns en suspension dans un liquide de viscosité supérieure à 0,2 mPa.s, ledit procédé permettant d'obtenir qu'au moins 99% en poids des particules entrantes et de diamètre supérieur à 20 microns, se retrouvent dans la partie inférieure dudit dispositif.

Le dispositif selon la présente invention peut être utilisé dans un procédé de séparation de fines particules de diamètre compris entre 10 et 100 microns en suspension dans un liquide de viscosité supérieure à 0,2 mPa.s, ledit procédé ayant une température de la suspension comprise entre 180°C et 250°C.

L'invention concerne également procédé de séparation de particules utilisant le dispositif selon la présente description.

Le dispositif selon la présente invention peut être utilisé dans un procédé de séparation de fines particules de diamètre compris entre 10 et 100 microns en suspension dans un liquide de viscosité supérieure à 0,2 mPa.s, ledit procédé permettant de réaliser un liquide concentré en particules solide (principalement situé dans la partie conique) avec une concentration supérieure d'au moins 10%, en valeur relative, par rapport à celle de la suspension introduite dans ledit dispositif.

Dans le cas de l'application du dispositif selon l'invention au procédé de synthèse Fischer Tropsch, le liquide concentré extrait par la tubulure (Ti) peut éventuellement être recyclé au réacteur de synthèse, préférentiellement au moyen d'une pompe.

L'invention concerne donc également de manière préférée un procédé de synthèse Fischer Tropsch mettant en oeuvre un réacteur de synthèse Fischer-Tropsch en mode "slurry" avec un catalyseur constitué de fines particules de diamètre compris entre 10 et 100 microns en suspension dans un liquide de viscosité supérieure à 0,2 mPa.s, dans lequel ledit procédé utilise un dispositif (décanteur) selon l'invention situé à l'extérieur du réacteur de synthèse et les particules extraites dudit dispositif par la tubulure inférieure (Ti) sont recyclées dans le réacteur au moyen d'une pompe.

### EXEMPLE (selon l'invention)

On traite une suspension issue d'un réacteur de synthèse Fischer Tropsch, constituée de particules de catalyseur, lesdites particules étant en suspension dans un liquide de masse volumique 720 kg/m3 et de viscosité 0,7 mPa.s, dans les conditions d'opération: température 200°C et pression de 25 bars (1 bar = 10⁵ pascal).

La masse volumique de la suspension (liquide + particules) à l'entrée du dispositif est de: 1100 kg/m3.

Le débit de suspension à traiter est de 115 tonnes /heures.

Les particules ont des diamètres compris entre 25 et 80 microns avec un diamètre moyen de 55 microns. Le dispositif selon l'invention a les dimensions suivantes:
Diamètre (D): 5,5 m
Hauteur de la partie cylindrique: 13,8 m
Hauteur de la partie conique: 2,8 m
Angle du cône (a): 45 °
Niveau d'introduction de la tubulure d'admission (h): 1,8 m
Diamètre de la tubulure d'admission dans sa partie horizontale (d1) :0,15 m
Diamètre de la tubulure d'admission dans sa partie verticale (d2) :0,0,38m
Diamètre de la tubulure d'évacuation du liquide clair (d3): 0,20m
Diamètre de la tubulure de soutirage de la suspension concentrée (d4): 0,15m
Angle d'inclinaison (β) du baffle intérieur: 50°
Hauteur du baffle intérieur : (h₁) : 0,75m
Hauteur de la portion verticale de la tubulure d'admission (hb): 1,5 m
Position du baffle: au niveau (hb) du début de la portion verticale de la tubulure d'admission (Ta)
La tubulure d'évacuation du liquide clair plonge de 0,5m au dessous du niveau de l'interface gaz-liquide.

La concentration en particules solides de la suspension en entrée est comprise entre 20 % et 30 % poids, et il a été montré par une méthode de simulation numérique reproduisant finement la géométrie du décanteur tel que défini plus haut, (et particulièrement la géométrie de la tubulure d'admission (Ta) en forme de coude), que la concentration dans le liquide soutiré par la tubulure (Ti) était augmenté de 7%, en valeur relative, par rapport à la concentration de la suspension entrante.

De plus, une mesure de la granulométrie d'un échantillon de la suspension récupéré au niveau de la tubulure de sortie (Ti) a montré que la proportion de particules ayant un diamètre supérieur à 20 microns est de 99,8% poids (contre 98% dans la suspension entrante).

## Revendications

1. Dispositif permettant la séparation de particules de solide de diamètre compris entre 10 et 100 microns dispersées sous forme d'une suspension dans un liquide, ledit dispositif comportant une partie supérieure de forme cylindrique de rapport H/D compris entre 2 et 6, et une partie inférieure conique de même diamètre maximal (D) que la partie cylindrique, l'angle (a) du cône étant compris entre 40° et 60°, ledit dispositif comprenant en outre :
- une tubulure d'admission (Ta) de la suspension en forme de coude, la portion horizontale dudit coude de diamètre d1 pénétrant à l'intérieur de la partie supérieure du dispositif, et la portion verticale dudit coude de diamètre d2 débouchant sensiblement au niveau du plan séparant la partie supérieure et la partie inférieure du dispositif, le rapport des diamètres d2/d1 étant compris entre 2 et 4, et la portion verticale de la tubulure d'admission (Ta) ayant une longueur (hb) égale à au moins 3 fois le diamètre d2 de ladite portion verticale,
- une tubulure de soutirage supérieure (Ts) du liquide clair située au sommet de la partie cylindrique supérieure,
- une tubulure de soutirage inférieure (Ti) des particules solides située en bas de la partie conique inférieure,
- un baffle intérieur (B), orienté vers le bas, faisant un angle (β) par rapport à la verticale, sensiblement égal au demi angle (α) du cône de la partie inférieure du dispositif, fixé à la paroi interne de la partie cylindrique du dispositif, à un niveau sensiblement identique au niveau du début de la portion verticale de la tubulure d'admission (Ta), la hauteur dudit baffle (B) étant comprise entre 0,4 et 0,6 fois la hauteur de la portion verticale de la tubulure d'admission (hb), et le diamètre (D) de la partie cylindrique étant compris entre 1 et 10 mètres.

2. Dispositif permettant la séparation de particules de solide selon la revendication 1, dans lequel le diamètre D du dispositif est compris entre 2 et 8 mètres.

3. Dispositif de séparation selon l'une quelconque des revendications 1 à 2, dans lequel la tubulure de soutirage du liquide clair (Ts) possède une portion verticale sensiblement centrée dans la partie supérieure et laquelle peut plonger à une profondeur comprise entre 0,2 et 1 mètre au dessous du niveau dudit liquide clair (H_{L}), et préférentiellement à une profondeur comprise entre 0,3 et 0,7 mètre au dessous dudit niveau (H_{L}).

4. Procédé de séparation de particules solide de diamètre compris entre 10 et 100 microns dispersées dans un liquide de viscosité supérieure à 0,2 mPa.s, utilisant le dispositif selon l'une quelconque des revendications 1 à 3.

5. Procédé de séparation selon la revendication 4, dans lequel le niveau liquide (H_{L}) est régulée automatiquement au moyen d'un contrôleur de niveau en agissant sur le débit de soutirage de liquide clair.

6. Procédé de séparation selon l'une quelconque des revendication 4 à 5, dans lequel la température de la suspension introduite dans le dispositif est comprise entre 180°C et 250°C.

7. Procédé de séparation selon quelconque des revendication 4 à 6, dans lequel le liquide concentré en particules solide situé dans la partie inférieure conique a une concentration supérieure d'au moins 10%, par rapport à la concentration de la suspension introduite dans le dispositif.

8. Procédé de séparation selon quelconque des revendication 4 à 7, dans lequel la vitesse d'écoulement du liquide dans la tubulure de soutirage (Ts) du liquide clair est comprise entre 0,1 et 0,3 m/s.

9. Procédé de séparation selon l'une quelconque des revendications 4 à 8, dans lequel la vitesse d'écoulement de la suspension dans la tubulure de soutirage inférieure (Ti) des particules solide est comprise entre 0,8 m/s et 3 m/s.

## Patentansprüche

1. Vorrichtung, welche die Trennung von Feststoffpartikeln mit einem Durchmesser zwischen 10 und 100 Mikron gestattet, die in der Form einer Suspension in einer Flüssigkeit verteilt sind, wobei die Vorrichtung einen oberen Teil mit zylindrischer Form mit einem Verhältnis H/D zwischen 2 und 6 und einen unteren konischen Teil mit demselben maximalen Durchmesser (D) wie der zylindrische Teil umfasst, wobei der Winkel (a) des Konus zwischen 40° und 60° liegt, wobei die Vorrichtung außerdem umfasst:
- ein Aufnahmerohr (Ta) der Suspension mit der Form einer Biegung, wobei der horizontale Abschnitt der Biegung mit dem Durchmesser d1 in das Innere des oberen Teils der Vorrichtung eindringt, und der vertikale Abschnitt der Biegung mit dem Durchmesser d2 im Wesentlichen auf der Höhe der Ebene einmündet, die den oberen Teil und den unteren Teil der Vorrichtung trennt, wobei das Verhältnis der Durchmesser d2/d1 zwischen 2 und 4 liegt, und der vertikale Abschnitt des Aufnahmerohrs (Ta) eine Länge (hb) gleich mindestens dem 3-Fachen des Durchmessers d2 des vertikalen Abschnitts aufweist,
- ein oberes Entnahmerohr (Ts) der klaren Flüssigkeit, das am Scheitel des oberen zylindrischen Teils angeordnet ist,
- ein unteres Entnahmerohr (Ti) der festen Partikel, das unten an dem unteren konischen Teil angeordnet ist,
- ein unteres Ablenkelement (B), das nach unten orientiert ist, das einen Winkel (β) in Bezug auf die Vertikale im Wesentlichen gleich dem halben Winkel (α) des Konus des unteren Teils der Vorrichtung bildet, und das an der Innenwand des zylindrischen Teils der Vorrichtung befestigt ist, auf einer Höhe, die im Wesentlichen identisch ist mit der Höhe des Beginns des vertikalen Abschnitts des Aufnahmerohrs (Ta), wobei die Höhe des Ablenkelements (B) zwischen dem 0,4- und 0,6-Fachen der Höhe des vertikalen Abschnitts des Aufnahmerohrs (hb) liegt, und wobei der Durchmesser (D) des zylindrischen Teils zwischen 1 und 10 Meter liegt.

2. Vorrichtung, welche die Trennung von Feststoffpartikeln ermöglicht, nach Anspruch 1, wobei der Durchmesser D der Vorrichtung zwischen 2 und 8 Meter liegt.

3. Trennvorrichtung nach einem der Ansprüche 1 bis 2, wobei das Entnahmerohr (Ts) der klaren Flüssigkeit einen vertikalen Abschnitt besitzt, der in dem oberen Teil im Wesentlichen zentriert ist und der in eine Tiefe zwischen 0,2 und 1 Meter unter den Pegel (H_{L}) der klaren Flüssigkeit eintauchen kann, und vorzugsweise in eine Tiefe zwischen 0,3 und 0,7 Meter unter den Pegel (H_{L}).

4. Verfahren zur Trennung von Feststoffpartikeln mit einem Durchmesser zwischen 10 und 100 Mikron, die in einer Flüssigkeit mit einer Viskosität von mehr als 0,2 mPa.s dispergiert sind, wobei die Vorrichtung nach einem der Ansprüche 1 bis 3 verwendet wird.

5. Trennverfahren nach Anspruch 4, wobei der Flüssigkeitspegel (H_{L}) automatisch mit einer Pegelsteuereinheit durch Einwirken auf den Durchsatz der Entnahme der klaren Flüssigkeit geregelt wird.

6. Trennverfahren nach einem der Ansprüche 4 bis 5, wobei die Temperatur der Suspension, die in die Vorrichtung eingebracht wird, zwischen 180°C und 250°C liegt.

7. Trennverfahren nach einem der Ansprüche 4 bis 6, wobei die mit Feststoffpartikeln konzentrierte Flüssigkeit, die in dem unteren konischen Teil angeordnet ist, eine Konzentration von mehr als mindestens 10 %, bezogen auf die Konzentration der Suspension, die in die Vorrichtung eingebracht wird, aufweist.

8. Trennverfahren nach einem der Ansprüche 4 bis 7, wobei die Fließgeschwindigkeit der Flüssigkeit in dem Entnahmerohr (Ts) der klaren Flüssigkeit zwischen 0,1 und 0,3 m/s liegt.

9. Trennverfahren nach einem der Ansprüche 4 bis 8, wobei die Fließgeschwindigkeit der Suspension in dem unteren Entnahmerohr (Ti) der Feststoffpartikel zwischen 0,8 m/s und 3 m/s liegt.

## Claims

1. A device allowing the separation of particles of solid with a diameter in the range 10 to 100 microns, dispersed in the form of a suspension in a liquid, said device comprising an upper portion which is cylindrical in shape with a ratio H/D in the range 2 to 6, and a lower conical portion having the same maximum diameter (D) as the cylindrical portion, the cone angle α being in the range 40° to 60°, said device further comprising:
• a tube (Ta) for admitting the suspension in the form of an elbow, the horizontal portion of said elbow, with diameter d1, penetrating into the interior of the upper portion of the device, and the vertical portion of said elbow, with diameter d2, opening substantially at the level of the plane separating the upper portion and the lower portion of the device, the ratio of the diameters, d2/d1, being in the range 2 to 4, and the vertical portion of the admission tube (Ta) having a length (hb) equal to at least three times the diameter d2 of said vertical portion;
• an upper withdrawal tube (Ts) for clear liquid located at the top of the upper cylindrical portion;
• a lower withdrawal tube (Ti) for solid particles located at the bottom of the lower conical portion;
• an internal baffle (B), downwardly orientated, making an angle (β) with respect to the vertical, which is substantially equal to the cone half angle (α) of the lower portion of the device, is fixed to the internal wall of the cylindrical portion of the device, at a level substantially identical to the level of the start of the vertical portion of the admission tube (Ta), the height of said baffle (B) being in the range 0.4 to 0.6 times the height of the vertical portion of the admission tube (hb), the diameter (D) being in the range 1 to 10 metres.

2. A device allowing separation of solid particles according to claim 1, in which the diameter D is in the range 2 to 8 metres.

3. A separation device according to claim 1 or claim 2, in which the clear liquid withdrawal tube (Ts) has a vertical substantially centred portion in the upper portion and which may be immersed to a depth in the range 0.2 to 1 metre below the level of said clear liquid (H_{L}), and preferably to a depth in the range 0.3 to 0.7 metres below said level (H_{L}).

4. A process for separating solid particles with a diameter in the range 10 to 100 microns dispersed in a liquid with a viscosity of more than 0.2 mPa.s, using the device according to any one of claims 1 to 3.

5. A separation process according to claim 4, in which the level of the liquid (H_{L}) is automatically adjusted using a level controller by acting on the withdrawal flow rate of the clear liquid.

6. A separation process according to claim 4 or claim 5, in which the temperature of the suspension introduced into the device is in the range 180°C to 250°C.

7. A separation process according to any one of claims 4 to 6, in which the liquid concentrated in solid particles located in the lower conical portion has a concentration which is at least 10% higher than the concentration of the suspension introduced into the device.

8. A separation process according to any one of claims 4 to 7, in which the flow rate of the liquid in the clear liquid withdrawal tube (Ts) is in the range 0.1 to 0.3 m/s.

9. A separation process according to any one of claims 4 to 8, in which the flow rate of the suspension in the lower solid particles withdrawal tube (Ti) is in the range 0.8 m/s to 3 m/s.
